# EUROPEAN PATENT SPECIFICATION

(11) **EP 0 841 880 B1**
(45) Date of publication and mention of the grant of the patent: **20.06.2001**
(21) Application number: 95906003.9
(22) Date of filing: 19.12.1994
(51) Int. Cl.: A61F 13/15

(54) **SANITARY NAPKIN HAVING A PLEATED LIFTING MEMBER**
HYGIENEVORLAGE MIT EINER GEFALTENEN AUFRICHTBAREN ZWISCHENSCHICHT
SERVIETTE HYGIENIQUE COMPRENANT UN ELEMENT DE SOULEVEMENT PLISSE

(30) Priority: 20.12.1993 US 170461; 08.04.1994 US 225405
(43) Date of publication of application: 20.05.1998
(73) Proprietor: THE PROCTER & GAMBLE COMPANY, Cincinnati, Ohio 45202 (US)
(72) Inventor: McFALL, Ronald R., West Chester, OH 45069 (US)
(74) Representative: Hirsch, Uwe Thomas
(86) International application number: US9414620
(87) International publication number: WO9517150

(56) References cited:
- EP-A- 0 426 197
- WO-A-92/07535

## Description

### FIELD OF THE INVENTION

The present invention relates generally to disposable absorbent articles such as sanitary napkins and, more particularly, to a sanitary napkin having a lifting member comprising a plurality of pleats for lifting and shaping a portion of the sanitary napkin.

### BACKGROUND OF THE INVENTION

Absorbent articles such as sanitary napkins, pantiliners, and incontinence pads are designed to absorb and retain liquid and other discharges from the human body, and to prevent soiling of the body and clothing by such discharges. It is generally desirable to provide absorbent articles such as sanitary napkins which maintain contact with the body of the wearer when they are worn, and which conform as closely as possible to the body of the wearer. Such body conforming capability is believed to increase the effectiveness of the sanitary napkin by reducing the possibility that menses will travel around the perimeter of the sanitary napkin and soil the wearer's body and/or clothing.

There have been a number of recent efforts to provide sanitary napkins and other absorbent articles with improved fit characteristics. Such recent efforts are described in U.S. Patent 4,950,264 issued August 21, 1990 to Osborn, U.S. Patent 5,007,906 issued April 16, 1991 to Osborn; U.S. Patent 5,197,959 issued March 30. 1993 to Buell; U.S. Patent Application Serial Number 07/605,583 entitled "Sanitary Napkin Having Components Capable of Separation In Use" filed October 29, 1990 in the name of Visscher et al.; and PCT International Publication Number WO 92/07535 published May 14, 1992 in the name of Visscher et al.

EP-B-426 197 discloses the use of arches (formed by a wick) which bend due to their resiliency in order to provide a shaping activity to sanitary napkins.

While the sanitary napkins disclosed in these references represent advancements in the art, the search for new and different ways of improving body contact has continued.

It is especially desirable that the sanitary napkin maintain contact with and conform to the body of the wearer under dynamic conditions (when the wearer walks, sits, etc.). For instance, when the sanitary napkin is put on, the sanitary napkin is subjected to lateral compression by the upper portions of the wearer's thighs. The forces applied by the wearer's thighs generally tend to distort the shape of the sanitary napkin, reducing the size of the target the sanitary napkin provides.

One attempt to control the effect of these compressive forces is disclosed in UK Patent Application 2,168,612A, published June 25, 1986. The UK patent application discloses a sanitary towel with a resilient insert positioned within the core or adjacent to a face of the core that is intended to inhibit permanent distortion of the towel. The UK application teaches that the insert resists lateral deformation of the sanitary towel, but does not teach or disclose a sanitary napkin having body conforming properties.

It is also desirable to provide a sanitary napkin which conforms to the wearer's body while maintaining the comfort of the wearer. Accordingly, a desirable sanitary napkin should maintain contact with the wearer's body, yet be capable of repeated elastic deflection to allow the wearer to comfortably assume different positions and to perform different activities.

In addition, sanitary napkins are generally fastened to the wearer's undergarments by adhesive or other means. Movement of the wearer's undergarments relative to the wearer's body can result in the sanitary napkin shifting from the desired position. It is therefore also desirable to provide a body conforming sanitary napkin with a mechanism to accommodate independent movement between the body of the wearer and the wearer's undergarments.

It is therefore an object of this invention to provide an absorbent article, such as a sanitary napkin, which intercepts menses by conforming to the shape of the female urogenital region.

It is another object of the present invention to provide a sanitary napkin having a convexly shaped body facing surface.

A further object of the present invention is to provide a sanitary napkin having a pleated lifting member disposed intermediate an absorbent core and a liquid impervious backsheet for providing repeated elastic displacement of the absorbent core and a liquid pervious topsheet relative to the backsheet.

These and other objects of the present invention will be more readily apparent when considered in reference to the following description and when taken in conjunction with the accompanying drawings.

### SUMMARY OF THE INVENTION

The present invention is an absorbent article, such as a sanitary napkin. The sanitary napkin of the present invention has a liquid pervious topsheet having a body facing surface, a liquid impervious backsheet joined to the topsheet, an absorbent core disposed intermediate the topsheet and the backsheet, and a longitudinally extending lifting member having a plurality of pleats along the length of the lifting member. The pleats have a Z-direction height for providing Z-direction displacement of a portion of the topsheet relative to the backsheet. The pleated lifting member can provide Z-direction elastic displacement of a portion of the topsheet relative to the backsheet, and convexly shape a portion of the body facing surface of the topsheet along a longitudinal centerline of the sanitary napkin.

The pleated lifting member can be nonabsorbent, and can comprise a first element and an elastic second element. The pleats can be formed by differentially elongating the elastic second element relative to the first element, and joining the first and second elements at spaced apart locations along their lengths. Contraction of the elastic second element gathers the first element to form pleats along the length of the first element. The pleats can be compressed by a Z-direction compressive load, such as when the wearer is sitting, to provide for the wearer's comfort. When the compressive Z-direction load is released, contraction of the elastic second element helps to restore the Z-direction height of the pleats, and thereby helps to maintain contact of the topsheet with the wearer's body.

In one embodiment the lifting member is disposed intermediate the absorbent core and the backsheet and extends along the longitudinal centerline of the disposable absorbent article. The lifting member has a first element, and an elastic second element disposed between the first element and the absorbent core. The second elastic element is joined to the first element at spaced apart locations along the length of the first element. The elastic second element contracts to gather the first element to form a plurality of pleats along the length of the first element. The lifting member can further include an elastic third element joined to the first element at spaced apart locations along the length of the first element. The elastic third element can be disposed intermediate the first element and the backsheet. The pleats of the first element extend between the second and third elastic elements and are stabilized by the second and third elastic elements, which prevent lateral collapse of the pleats.

The Z-direction height of the pleats can vary along the longitudinal axis of the sanitary napkin. In one embodiment, the Z-direction height of the pleats is maximum along a center portion of the lifting member, which center portion can be generally aligned with the lateral centerline of the sanitary napkin. The Z-direction height decreases forward and rearward of the center portion. In a second embodiment the Z-direction height of the pleats can be tapered to have a maximum Z-direction height rearward of a lateral centerline of the sanitary napkin and a minimum Z-direction height forward of the lateral centerline of the sanitary napkin.

### BRIEF DESCRIPTION OF THE DRAWINGS

Figure 1 is a top plan view of the sanitary napkin of the present invention with portions of the sanitary napkin shown cut away.

Figure 2 is a section view taken along line 2-2 of Figure 1 which shows the sanitary napkin of the present invention in a compressed configuration.

Figure 3 is a section view similar to that of Figure 2 showing the sanitary napkin in an extended configuration.

Figure 4 is a section view taken along line 4-4 of Figure 1 which shows a sanitary napkin of the present invention in an extended configuration, and a lifting member with pleats of generally uniform Z-direction height, the pleats extending along the longitudinal centerline of the sanitary napkin.

Figure 5 is a section view similar to that of Figure 4 showing a lifting member having pleats with a maximum Z-direction height along a center portion of the lifting member, which center portion is generally aligned with the lateral centerline of the sanitary napkin.

Figure 6 is a section view similar to that of Figure 4 showing a lifting member having pleats with a Z-direction height that tapers from a maximum value rearward of the lateral centerline of the sanitary napkin to a minimum value forward of the lateral centerline of the sanitary napkin.

Figure 7 is a schematic illustration of a method for forming the lifting member of the present invention by differentially elongating two or more elements and intermittently joining the differentially elongated elements along their lengths.

Figure 8 is a top plan view of a sanitary napkin having a lifting member having pleats with a Z-direction height and lateral displacement members having pleats with a lateral height.

### DETAILED DESCRIPTION OF THE INVENTION

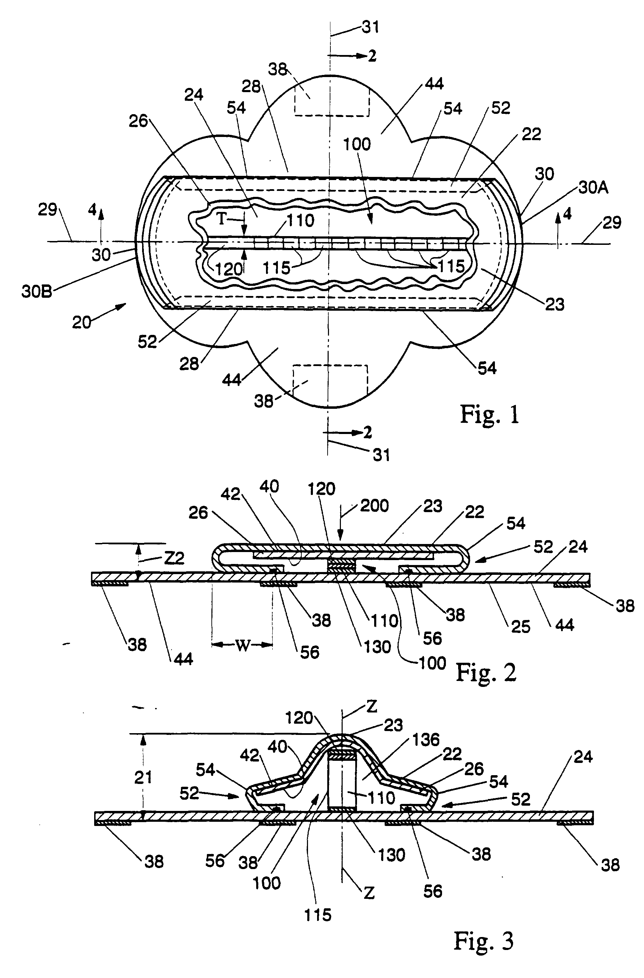

Figures 1-4 illustrate a sanitary napkin 20 according to one embodiment of the disposable absorbent article of the present invention. As used herein, the term "absorbent article" refers to articles which absorb and contain body exudates. More specifically, the term is intended to include, but not be limited to, sanitary napkins, pantiliners, and incontinence pads (articles worn in the crotch region of a garment). The term "disposable" refers to articles which are intended to be discarded after a single use rather than laundered or otherwise restored or reused.

The sanitary napkin 20 has two longitudinal ends 28, and two lateral ends 30. The lateral ends 30 can comprise a forward lateral end 30A and a rearward lateral end 30B. The sanitary napkin also has a longitudinal centerline 29 and a lateral centerline 31. As used herein the term "longitudinal" refers to a line, axis, or direction generally aligned with the vertical plane which bisects the standing wearer into left and right body halves. The term "lateral" refers to a line, axis, or direction generally perpendicular to the longitudinal direction and lying within the plane of the sanitary napkin 20. The sanitary napkin 20 is typically longer in the longitudinal direction than in the lateral direction.

The "Z" direction refers to a line, axis, or direction which is perpendicular to the plane of the sanitary napkin (i.e., perpendicular to both the longitudinal axis 29 and the lateral axis 31 when the sanitary napkin is supported in a generally flat configuration). The Z direction is illustrated in Figure 3.

The sanitary napkin 20 comprises a liquid pervious topsheet 22 having a body facing surface 23, a liquid impervious backsheet 24 having a garment facing surface 25, an absorbent core 26 intermediate the topsheet 22 and the backsheet 24, and a lifting member 100, which can be disposed intermediate the topsheet 22 and the backsheet 24.

The lifting member 100 comprises a plurality of pleats 115. The pleats 115 provide Z-direction elastic displacement of a portion of the topsheet 22 along the longitudinal centerline 29, and preferably a portion of the absorbent core 26, relative to the backsheet 24. The pleats 115 also preferably convexly shape a portion of the body facing surface 23 of the topsheet 22 along the longitudinal centerline 29, as shown in Figure 3. The lifting member 100 thereby maintains contact of the topsheet 22 with the wearer's body, and shapes the topsheet 22 to conform to the wearer's body, particularly in the labia, perineum, and/or gluteal groove areas.

The lifting member 100 can be disposed intermediate the backsheet 24 and the absorbent core 26 and preferably elastically displaces and shapes both the topsheet 22 and the core 26. At least a portion of the core 26 is thereby biased into contact with the topsheet 22 to receive body exudates passing through the liquid pervious topsheet 22. The lifting member 100 can extend between the core 26 and the backsheet 24 and preferably lifts the core 26 from the backsheet 24 to provide a void space 136. The void space 136 extends in the Z-direction from the backsheet 24 to the absorbent core 26. The void space 136 is desirable to ensure that the lifting member 100 is the only element providing resistance to displacement of the topsheet 22 and core 26 toward the backsheet 24, such as by a compressive load 200. Alternatively, an absorbent material such as airfelt can partially or completely fill the space between the backsheet 24 and the absorbent core 26.

The lifting member 100 can comprise a longitudinally extending first pleated element 110 having a plurality of pleats 115 along its length. Each pleat 115 has a Z-direction height when the sanitary napkin 20 is in the extended position shown in Figure 3. The pleats 115 can provide Z-direction elastic displacement of the topsheet 22 and the core 26 relative to the backsheet 24. The lifting member 100 preferably also includes a longitudinally extending second element 120 disposed intermediate the first element 110 and the absorbent core 26. The lifting member 100 can also include a longitudinally extending third element 130 disposed intermediate the first element 110 and the backsheet 24. The second and third elements 120 and 130 can be joined to the first member 110 at spaced apart locations along their respective lengths. The pleats 115 extend between the second and third members 120,130. The second and third members 120 and 130 bridge adjacent pleats 115 and stabilize the pleats 115 to help prevent lateral collapse (such as by buckling) of the pleats 115 when the sanitary napkin is in the extended position shown in Figure 3.

The second element 120 preferably comprises an elastic second element 120. Elastic contraction of the elastic second element 120 gathers the first element 110 about fold lines generally parallel to the lateral axis 31 to form the pleats 115 along the length of the first element 110. The second element 120 is also preferably elastic to provide a force for maintaining the Z-direction height of the pleats 115, and for restoring the Z-direction height of the pleats 115 when the pleats 115 are compressed by a Z-direction load such as the compressive load 200 shown in Figure 2. The pleats 115 are compressed by the load 200, such as when the wearer is sitting, to provide for the comfort of the wearer. When the compressive load 200 is removed (e.g. when the wearer stands up), contraction of the elastic second element 120 restores the Z-direction height of the pleats 115 and thereby maintains contact of the topsheet 22 with the wearer's body.

The third element 130 can also comprise an elastic third element 130. In one embodiment both of the second and third elements 120 and 130 are elastic elements, and elastic contraction of one or both of the elastic second and third elements 120, 130 gathers the first elastic element 110 to form the pleats 115. In yet another embodiment the first element 110 can also comprise an elastic element.

By "Z-direction elastic displacement" of the topsheet 22 relative to the backsheet 24, it is meant that the topsheet 22 can be displaced relative to the backsheet 24 in the Z-direction from a first, extended configuration having a Z-direction caliper Z1 shown in Figure 3, to a second compressed configuration having a caliper Z2 shown in Figure 2 (such as by the compressive Z-direction load 200), and that the lifting member 100 will restore the sanitary napkin 20 to have a Z-direction caliper which is at least about 70 percent of the Z-direction caliper Z 1 upon release of the compressive loading, when the sanitary napkin is dry and has not been loaded with body exudates. The elastic displacement of the topsheet 22 relative to the backsheet 24 can be expressed by the difference Z1 - Z2.

The term "elastic element" refers to a component which has a free length, and which can be strained by a tensile force to have a percentage of elongation of at least 35 percent (elongated length greater than or equal to 1.35 x free length), and wherein upon release of the tensile force the component contracts to within 5 percent of its free length within ten seconds.

The topsheet 22 and the backsheet 24 are joined together adjacent the longitudinal ends 28 and along one or both of the lateral ends 30. As used herein the term "join" refers to the condition where a first member or component is attached or connected to a second member or component either directly; or indirectly, where the first member or component is attached, or connected, to an intermediate member or component which in turn is attached, or connected to the second member or component.

Examining the components of the sanitary napkin 20 in more detail, the topsheet 22 is the component of the sanitary napkin 20 oriented towards and contacting the body of the wearer for receiving body exudates. The topsheet 22 is flexible, soft feeling, non-irritating to the wearer's skin, and is liquid pervious. As used herein, the term flexible refers to materials which are compliant and readily conform to the shape of the body or respond by easily deforming in the presence of external forces. Preferably, the topsheet 22 is not noisy to provide discretion to the wearer. The topsheet 22 should be clean in appearance and can be somewhat opaque to hide the discharges collected in the core 26.

The topsheet 22 should exhibit good strike-through and rewet characteristics, permitting bodily discharges to rapidly penetrate the topsheet 22 to the core 26. A suitable topsheet 22 may be made from a wide range of materials, such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or combinations of natural and synthetic fibers.

The topsheet 22 has a plurality of apertures to permit liquids deposited thereon to pass through to the core 26. The topsheet 22 can comprise an apertured formed polyolefinic film having about 5 to about 60 percent open area and a thickness of about 0.01 to about 0.05 millimeters. If desired, the topsheet 22 can be sprayed with a surfactant to enhance fluid penetration to the core 26. A suitable surfactant is sold by Glyco Chemical Inc. of Greenwich, Connecticut as Pegosperse 200 ML.

A suitable topsheet 22 may be made in accordance with U.S. Patent 3,929,135 issued December 30, 1975 to Thompson; U.S. Patent 4,324,246 issued April 13, 1982 to Mullane; U.S. Pat. No. 4,342,314 issued Aug. 3, 1982 to Radel et al.; U.S. Pat. No. 4,463,045 issued July 31, 1984 to Ahr et al.; and U.S. Patent 5,006,394 issued April 9, 1991 to Baird, which patents disclose particularly preferred executions of liquid pervious topsheets. Each of these patents are incorporated herein by reference. A suitable topsheet 22 for the present invention is the formed film described in one or more of the above patents and marketed on sanitary napkins by The Procter and Gamble Company of Cincinnati, Ohio as "DRI-WEAVE." An elastically extensible topsheet 22 made of Model X-3265 or Model P1552 apertured formed film sold by the Ethyl Corp., Visqueen Division, of Terre Haute, Indiana can also be used.

The backsheet 24 may be any flexible liquid impervious material, such as a polyolefinic film. The backsheet 24 prevents discharges collected by the sanitary napkin 20 from soiling the wearer or the wearer's clothing. The backsheet 24 can be a low density polyethylene film about 0.01 to about 0.05 millimeters in thickness. Suitable polyethylene films from which the backsheet 24 can be formed are sold by the Ethyl Corp., Visqueen Division, as Model XP-39385 and by the Clopay Corp. of Cincinnati, Ohio as P18-1401.

The backsheet 24 can have a surface area greater than or equal to that of the topsheet 22 and the absorbent core 26, and preferably peripherally circumscribes the topsheet 22 and the core 26. The backsheet 24 may comprise flaps 44 extending outwardly from each longitudinal edge 28. The flaps 44 may be made in accordance with the teachings of U.S. Patent Nos. 4,589,876 issued May 20, 1986 to Van Tilburg and 4,687,478 issued August 18, 1987 to Van Tilburg. The backsheet 24 and the flaps 44 may be unitary and coextensive. Alternatively, the flaps 44 can be separate components joined to the backsheet 24.

The garment facing surface 25 of the backsheet 24 may comprise an attachment means 38 for securing the sanitary napkin 20 to the undergarment of the wearer. Preferred attachment means 38 include mechanical fasteners, or more preferably, pressure sensitive adhesive 38. The pressure sensitive adhesive 38 may be applied to the garment facing surface 25 in one or more strips or patches. As shown in Figures 1 and 2, the pressure sensitive adhesive can be disposed near the distal end of each flap 44, as well as on a portion of the backsheet 24 underlying the topsheet 22 and absorbent core 26. A suitable adhesive 38 is Century Adhesive A-305-IV manufactured by Century Adhesives Corp. of Columbus Ohio.

The absorbent core 26 receives and contains body exudates, particularly menses. The core 26 should be nonirritating to the skin, and may be manufactured in a wide variety of shapes (e.g., rectangular, oval, hourglass, dog bone, asymmetric, etc.), sizes and thicknesses. The core 26 has a first face 40 oriented towards the backsheet 24, and a second opposed face 42 oriented towards the topsheet 22.

Suitable materials from which the core 26 can be made include but are not limited to combinations of airfelt, such as cellulose wadding, and fibrated communition pulp; meltblown polymers, chemically stiffened, modified, or crosslinked cellulosic fibers; absorbent foams; layers of tissue paper; and absorbent gelling materials. The core 26 can comprise a laminate of tissue paper and absorbent gelling material. A suitable core 26 can comprise two layers of tissue paper as disclosed in U.S. Patent 5,007,906 issued April 16, 1991 to Osborn et al., which patent teaches a suitable construction for core 26. Other exemplary absorbent structures for use as the absorbent core of the present invention are described in U.S. Patent 4,950,264 issued August 21, 1990 to Osborn; U.S. Patent 4,610,678 issued September 9, 1986 to Weisman et al.; U.S. Patent 4,834,735 issued May 30, 1989 to Alemany et al.; and European Patent Application No. 0 198 683 published October 22, 1986 in the name of Duenk et al..

In one embodiment the core 26 and the topsheet 22 are joined together to form a laminate so that the core 26 and the topsheet 22 can be displaced by the lifting member 100 as a unit. The second face 42 of the core 26 can be joined to the topsheet 22 by any suitable means. such as by adhesive attachment. Such integration of the topsheet 22 with the absorbent core 26 maintains contact between the topsheet 22 and the core 26 during wear, and provides capillary suction of the fluids passing through the topsheet 22 into the core 26.

The sanitary napkin 20 according to the present invention can have the core 26 and the associated topsheet 22 decoupled from the backsheet 24 such that the topsheet 22 is joined to the backsheet 24 to provide independent Z-direction movement of the topsheet 22 and the core 26 relative to the backsheet 24. A suitable sanitary napkin construction for providing such Z-direction decoupled motion of the topsheet 22 and the core 26 relative to the backsheet 24 is disclosed in U.S. Patent 5,007,906 issued April 16, 1991 to Osborn et al..

Such decoupling is desirable to permit the topsheet 22 and the core 26 to be elastically displaced by the lifting member 100 into contact with the wearer's body, while allowing the backsheet 24 to remain anchored to the wearer's garment by the attachment means 38.

The sanitary napkin 20 can have a means for controlling the amount of Z-direction separation of the topsheet 22 and the associated core 26 from the backsheet 24. One suitable means for providing such control is one or more longitudinally extending folds 52 which form a connection joining the topsheet 22 to the backsheet 24. As used herein a "longitudinally extending fold" is a component of the sanitary napkin 20 having a longitudinally extending fold line 54 to provide one or more Z-direction layers of material along the fold line 54. Preferably two longitudinally extending folds 52 are provided, one at each longitudinal end 28 of the sanitary napkin 20.

The longitudinally extending fold 52 may be an extension of the topsheet 22. an extension of the backsheet 24, or a separate piece of material having one end joined to the topsheet 22 and one end joined to the backsheet 24. The fold 52 can be formed by folding a portion of the topsheet 22 along fold line 54 and joining the free edge of the folded portion of the topsheet to the backsheet 24 along bond lines 56 Bond lines 56 can comprise heat sealing or adhesive bond lines. Bond lines 56 are preferably continuous to form a seal between the topsheet 22 and the backsheet 24, and can comprise lines of adhesive bonding between the topsheet 22 and the backsheet 24.

The bond lines 56 and the fold lines 54 can be generally parallel, as shown in Figure 1, to accommodate a equal amount of Z-direction displacement along the length of the sanitary napkin 20. Alternatively, the bond lines 56 or the fold lines 54 can diverge or converge along the length of the sanitary napkin 20, to accommodate different amounts of Z-direction displacement of the topsheet 22 and core 26 along the length of the sanitary napkin 20.

The folds 52 shown in Figures 1-3 are extensions of the topsheet 22 and comprise a single fold line 54 to form a C-shaped fold. Alternatively, accordion-shaped folds having a plurality of fold lines 54 can be used. Above referenced U.S. Patent 5,007,906 is incorporated herein by reference for the purpose of describing longitudinally extending folds 52.

The topsheet 22 may be left unattached to the backsheet 24 at one of the lateral ends 30, such as rear lateral end 30B, to further accommodate Z-direction decoupling of the topsheet 22 and core 26 from the backsheet 24. Additionally, leaving the topsheet 22 unattached to the backsheet at one of the lateral ends 30 also accommodates decoupling of the topsheet 22 and core 26 from the backsheet 24 in the longitudinal direction. Such longitudinal decoupling permits relative movement of the topsheet 22 and core 26 with respect to the backsheet 24 (and the wearer's undergarment to which the backsheet is attached) in the plane of the sanitary napkin 20. Alternatively, the topsheet 22 can be joined to the backsheet at one of the lateral ends 30 by a laterally extending fold (not shown) to provide longitudinal decoupling of the topsheet 22 and core 26 with respect to the backsheet 24 in the plane of the sanitary napkin 20.

In one embodiment, the ability of the lifting member 100 to restore the Z-direction caliper of the sanitary napkin 20 is relatively unaffected by wetting of the lifting member 100. The lifting member 100 can have a wet caliper reduction which is no more than about 20 percent greater than its dry caliper reduction, and a wet caliper reduction of no more than about eight percent. The wet caliper reduction and dry caliper reduction for the lifting member 100 are measured using the following procedure repeated for four lifting member samples.

The lifting member 100 is adhesively attached to a sheet of polyethylene film having a thickness of about 1.0 mil. The lifting member 100 and polyethylene film are supported on the horizontal surface of an analytical balance, or other suitable scale. The Z-direction caliper of the lifting member 100 above the polyethylene film is measured using a suitable displacement measuring system. A suitable displacement measuring system is an ONO-SOKKI DG 3610 Digital Gauge and an ONO-SOKKI GS-503 Linear Gauge Sensor available from the ONO-SOKKI Corporation of Japan. The Z-direction caliper of the lifting member 100 is measured at various Z-direction load levels applied to the lifting member 100 through a circular load application foot having a diameter of 0.95 inch. The load application foot is connected to the linear gauge sensor.

The lifting member 100 and polyethylene film are placed on the balance, and the balance is tared out to have a zero reading. The initial dry Z-direction caliper of the lifting member 100 is measured with the load application foot just touching the lifting member 100, so that the balance indicates a reading of about zero. The Z-direction load on the lifting member 100 is increased to 32.1 grams in about 5 equal increments, so that the balance indicates a weight of 32.1 grams. The load is then removed, and the unloaded dry Z-direction caliper of the lifting member 100 is recorded with the load application foot just touching the lifting member 100, so that the balance indicates a reading of about zero. For each sample, the difference between the initial dry Z-direction caliper and the unloaded dry Z-direction caliper is divided by the initial dry Z-direction caliper to obtain the percentage change in dry caliper of the sample. The dry caliper reduction is the average of the percentage change in dry caliper for the four lifting member samples.

Each lifting member (and its associated polyethylene sheet) is completely submerged in distilled water for 10 seconds, and then allowed to drain vertically for 10 seconds. The lifting member 100 and polyethylene sheet are then supported on the horizontal surface of the analytical balance, and the balanced tared out to indicate a reading of zero. The initial wet Z-direction caliper of the lifting member 100 is measured with the load application foot just touching the lifting member 100, so that the balance indicates a reading of about zero. The Z-direction load on the lifting member 100 is then increased to 32.1 grams in about 5 equal increments. The load is then removed and the unloaded wet Z-direction caliper of the lifting member 100 is recorded with the load application foot just touching the lifting member 100, so that the balance indicates a reading of about zero. For each sample, the difference between the initial wet Z-direction caliper and the unloaded wet Z-direction caliper is divided by the initial wet Z-direction caliper to obtain the percentage change in the wet caliper of the sample. The wet caliper reduction of the lifting member 100 is the average of the percentage change in wet caliper for the four lifting member samples.

In one embodiment, the lifting member 100 is nonabsorbent. By "nonabsorbent" it is meant that the lifting member 100 has an absorbency capacity of less than 100 percent. The absorbency capacity is the ratio of the weight of the water absorbed by a dry sample to the dry sample weight. A nonabsorbent lifting member 100 is believed to have the advantage that its stiffness and/or its ability to displace the core upward are relatively unaffected by body fluids entering the sanitary napkin 20, as compared to a lifting member which is absorbent. The absorbency capacity of the lifting member is measured by first weighing the lifting member 100 to obtain its dry weight, and then completely submerging the lifting member 100 in distilled water for 10 seconds. After 10 seconds the lifting member 100 is removed from the water. The lifting member is then allowed to drain vertically for 10 seconds. Water adhering to the surface of the lifting member is then removed by blotting the lifting member between two pieces of filter paper for 10 seconds. The lifting member 100 is blotted by placing a first piece of filter paper on a dry horizontal surface, placing the lifting member on the first piece of filter paper, placing a second piece of filter paper on top of the lifting member to cover the lifting member, and placing a piece of 0.25 inch thick Plexiglas weighing 0.26 pound on top of the second piece of filter paper to cover the portion of the second piece of filter paper overlying the lifting member. A suitable filter paper for blotting the lifting member 100 is filtration paper having a relatively smooth surface, a particle retention size of greater than about 20-25 micrometers, and a Herzberg filtration speed of about 37 seconds, where the filtration speed is the time for 100 ml of prefiltered water to pass through a 10.0 square centimeter piece of filter paper with a constant head pressure of 10 centimeters of water. A suitable filtration paper is Whatman 4 filtration paper manufactured by Whatman Ltd. of England and available from the Fisher Scientific Company of Pittsburgh, Pa. After blotting the lifting member 100 for 10 seconds, the lifting member 100 is immediately weighed to obtain the wet sample weight. The dry weight is subtracted from the wet weight to yield the grams of water absorbed by the dry sample. The percentage absorbency capacity is obtained by dividing the grams of water absorbed by the dry sample weight, and multiplying the quotient by 100.

In a preferred embodiment the lifting member 100, and particularly the pleats 115, is hydrophobic. A surface is hydrophobic if the contact angle between a liquid and the surface is greater than 90 degrees. The American Chemical Society Publication "Contact Angle, Wettability, and Adhesion," edited by Robert F. Gould and copyrighted in 1964 is incorporated herein by reference for the purpose of showing how the contact angle can be determined.

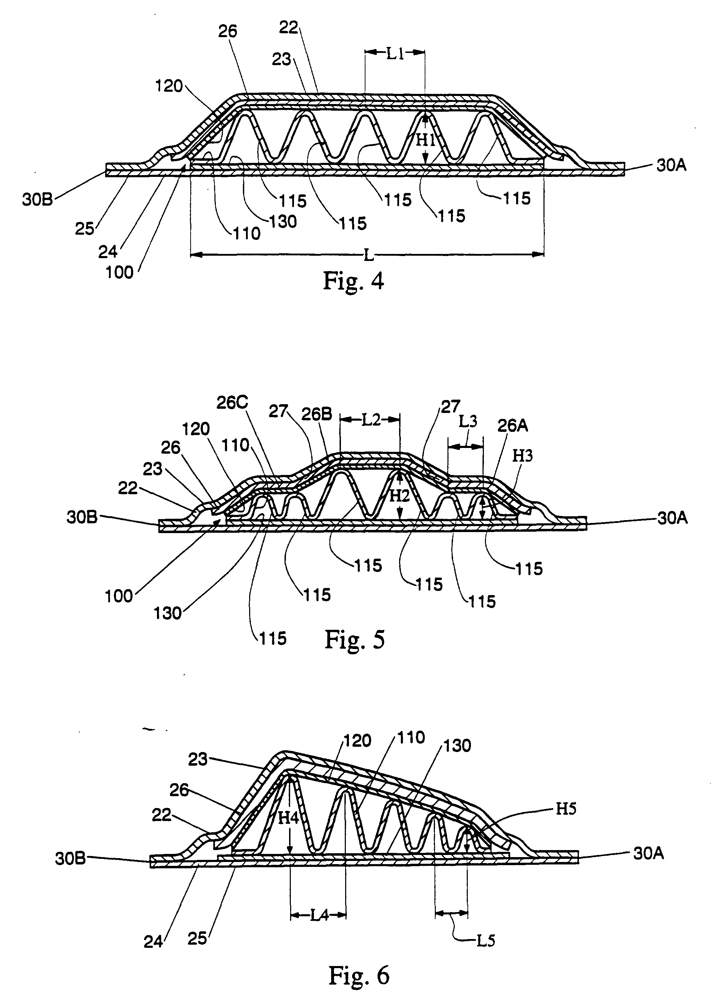

In a preferred embodiment the lifting member 100 extends along the longitudinal centerline 29 of the sanitary napkin 20, and can have a longitudinal length L (Figure 4) which is greater than its lateral width T (Figure 1). The lateral width T is preferably less than the lateral width of the core 26, and in one embodiment is preferably less than about 1/4 the lateral width of the core 26. A narrow lateral width T is desirable for close fit of the body facing surface 23 with the labia, perineum, and/or gluteal groove areas of the body along the longitudinal centerline 29 of the sanitary napkin 20. In one embodiment, the lateral width T is preferably no more than about 10.0 mm, and more preferably not more than about 6.0 mm. The longitudinal length L can be between about 150 and about 250 mm. In an alternative embodiment, the lateral width T can be greater than 10.0 mm.

The array of pleats 115 extends along the length L of the lifting member 100. Each pleat 115 has a Z direction height (H1 in Figure 4). The lifting member 100 can thereby provide Z-direction displacement of the portion of the topsheet 22 and absorbent core 26 relative to the backsheet 24 along the longitudinal centerline 29. The lifting member 100 can also convexly shape the body facing surface 23 of the topsheet 22, as shown in Figure 3, to form a longitudinally extending ridge along the longitudinal centerline 29 of the sanitary napkin 20.

In the embodiments shown the lifting member 100 comprises a first element 110, an elastic second elastic element 120, and an elastic third elastic element 130. The pleats 115 of the first element 110 extend between the elastic second element 120 and the elastic third element 130. The second and third elastic elements 120, 130 bridge adjacent pleats 115 and help to prevent lateral collapse of the pleats 115 (such as by buckling) when the sanitary napkin is in the extended configuration shown in Figure 3, while permitting the pleats 115 to be compressed by a Z direction force 200 (e.g., a body force) as shown in Figure 2.

The second element 120 can be joined to the surface 40 of the core 26, and the third element 130 can be joined to the backsheet 24. In alternative embodiments one or both of the second and third elements 120 and 130 can be omitted so that the first element 110 is joined directly to the core 26 and/or the backsheet 24.

The first element 110 can be formed from a number of suitable materials, including but not limited to woven and nonwoven sheet material, plastic films, and natural or synthetic rubber strands. One suitable material from which the first element 110 can be formed is a polypropylene mesh scrim material having a basis weight of about 100 grams per square meter and available as P100 polypropylene mesh scrim from Smith and Nephew Plastics, Ltd. of Gilberdyke, North Humberside, UK. A sheet of such a mesh scrim has about 40 relatively thicker and stiffer primary strands per centimeter (16 strands per inch) running in the sheet machine direction and about 61 relatively thinner and less stiff secondary strands per centimeter (24 strands per inch) running perpendicular to the primary strands in the sheet cross machine direction. The first element 110 can comprise a 6.0 mm wide strip of the P100 mesh cut parallel to the primary strands, so that the primary strands extend along the length of the first element 110, and generally parallel to the longitudinal axis 29 of the sanitary napkin 20. Another suitable material from which the first element 110 can be formed comprises a polypropylene mesh scrim having a basis weight of about 50 grams per square meter (10 lbs/1000 square feet) available as ON7100 polypropylene mesh from the Conwed Company of Minneapolis, Minnesota.

The elastic second and third elements 120 and 130 can be formed from a number of types of elastic material including natural or synthetic rubber strands, elastic woven or nonwoven materials, and elastic films. One suitable material from which the second and third elements 120 and 130 can be formed is an elastic tape sold by Fulflex, Inc. of Middletown, Rhode Island as ULTRAFLEX MODEL 6EX29 elastic tape. The second and third elements 120 and 130 can each comprise a length of such an elastic tape having a width of between about 4.0 and 6.0 mm and a thickness of about 2.0 mm. Alternatively, the elements 120 and 130 can each comprise a 6.0 mm wide strip of EXX-500 elastic sheet material available from the Exxon Chemical Company of Buffalo Grove, Illinois.

The pleats 115 in the first element 110 can be formed by elastic contraction of one or both of the second and third elements 120,130 relative to the first element 110. For instance, the first element 110 can have a free (unstretched) length which is greater than the free length of the second element 120 and the third element 130. The second and third elements 120 and 130 can be elongated relative to their free lengths and relative to the first element 110. While elongated, the first and second elements 120 and 130 can be attached to the first element 110 at spaced apart locations along the length of the first element 110. When the forces causing the elongation of the elastic elements 120 and 130 are released, the elastic elements 120 and 130 will contract relative to the first element 110, thereby drawing the spaced apart attachment points on the first element 110 together to form the pleats 115.

Percentage elongation is determined by subtracting an elongated length from the free gage length, and dividing the difference by the free gage length. For elastic elements 120 and 130 formed from the ULTRAFLEX elastic tape listed above, a suitable lifting member 110 with pleats 115 can be made by providing a percentage of elongation in the second and third elements 120 and 130 of between about 35 and about 400 percent. The second and/or third elastic elements 120 and 130 can be attached to the first element 110 at locations spaced apart a distance of between about 25.4 mm (1.0 inch) and about 127 mm (5.0 inch) as measured when the elastic elements are elongated and prior to gathering of the first element 110 by contraction of the second and third elements 120 and 130.

In the embodiment shown in Figure 4 the Z-direction height H1 of the pleats 115 is generally uniform along the length L of the lifting member 100. By way of example, the height H1 can be between about 20 mm to about 40 mm, and the spacing L1 between adjacent pleats 115 can be between about 25 mm to about 50 mm. For a lifting member 100 having a first element formed from the P100 mesh scrim material listed above and having first and second elastic elements 120, 130 formed from the ULTRAFLEX elastic tape listed above, such a uniform arrangement of pleats 115 can be obtained where the percentage of elongation in the second and third elements 120 and 130 is about 50 to about 400 percent and where the second and third elements 120 and 130 are attached to the first element 110 at locations spaced apart a distance of about 25.4 mm to about 127 mm as measured when the second and third elements 120 and 130 are elongated and prior to gathering the first element 110 by contraction of the second and third elements 120 and 130.

The Z-direction height of the pleats 115 can vary along the longitudinal length L of the lifting member 100. In particular, the Z-direction height of the pleats 115 can vary along the longitudinal axis 29 of the sanitary napkin 20, such as when the lifting member 100 extends along the axis 29. Such a variation in Z-direction height can provide localized lift of the topsheet 22 and core 26 for improved fit in the labia, perineum, and/or gluteal groove areas of the body. Such a variation in Z-direction height of the pleats 115 can be obtained by varying the percentage of elongation of the second and/or third elements 120, 130 as a function of position along the length of the first elastic element 110 prior to joining the elastic elements together.

In the embodiment shown in Figure 5, the Z-direction height of the pleats 115 has a maximum value of H2 along a center portion of the length L of lifting member 100, which center portion can extend longitudinally across the lateral centerline 31 of the sanitary napkin. The Z-direction height has a lower value H3 in forward and rearward end portions of the length L. By way of example, the height H2 can be between about 20 mm and about 40 mm with spacing L2 between adjacent pleats 115 of between about 25 mm and about 50 mm, and the height H3 can be between about 10 mm and about 20 mm with spacing L3 between adjacent pleats 115 of between about 10 mm and about 25 mm. Such a variation in Z-direction height of the pleats 115 can be obtained with a first element 110 formed of the P100 scrim mesh and second and third elements 120 and 130 formed of the ULTRAFLEX elastic tape, as described above, and with the following elongations and spacing: In the center portion, the second and third elements 120 and 130 are stretched to an elongation of about 200 percent and are attached to the first element 110 at points spaced apart about 100 mm (4.0 inch) along the length of the first element 110; in the forward and rearward end portions, the second and third elements are stretched to an elongation of about 200 percent and are attached to the first element 110 at points spaced apart about 50 mm (2.0 inch) along the length of the first element 110.

In another embodiment shown in Figure 6, the Z-direction height of the pleats 115 can be tapered to have a maximum Z-direction height H4 rearward of the lateral centerline 31 of the sanitary napkin 20 and a minimum Z-direction height H5 forward of the lateral centerline 31. The height H4 can be between about 30 mm and about 40 mm and the height H5 can be between about 10 mm and about 20 mm. The spacing between adjacent pleats 115 can vary from a spacing L4 rearward of the lateral centerline 31 of between about 25 mm and about 50 mm to a spacing L5 forward of the lateral centerline 31 of between about 10 mm and about 25 mm. Such a variation in Z-direction height of the pleats 115 can be obtained with a first element 110 formed of the P100 scrim mesh and second and third elements 120 and 130 formed of the ULTRAFLEX elastic tape, as described above, and with the following elongations and spacing: in the forward portion, the second and third elements 120 and 130 are stretched to an elongation of about 200 percent and are attached to the first element 110 at points spaced apart about 50 mm (2.0 inch) along the length of the first element 110; in center portion, the second and third elements are stretched to an elongation of about 300 percent and are attached to the first element 110 at points spaced apart about 50 mm (2.0 inch) along the length of the first element 110; in the rearward portion, the second and third elements 120 and 130 are stretched to an elongation of about 200 percent and are attached to the first element 110 at points spaced apart about 100 mm (4.0 inch) along the length of the first element 110.

The lifting member 100 preferably has pleats 115 having a Z-direction height of at least about 10 millimeters, and preferably at least about 15 millimeters. The lifting member 100 preferably provides a Z-direction elastic displacement Z1-Z2 of at least 10 millimeters, and more preferably of at least 15 millimeters so that the topsheet 22 can conform to the wearer's body while accommodating relative motion between the wearer's undergarments and the wearer's body. The lifting member 100 can provide such Z-direction elastic displacement with a relatively low force per unit displacement so that the topsheet 22 comfortably conforms to the wearer's body.

Referring to Figures 2 and 3, the lifting member 100 can provide the sanitary napkin 20 with a first Z-direction caliper Z1 under a compressive load of 2 grams, and a second Z-direction caliper Z2 which is at least 10 mm less than the first Z-direction caliper Z1, under a compressive load less than 50 grams, and preferably less than 30 grams. The lifting member 100 can also provide the sanitary napkin 20 with a second Z-direction caliper Z2 which is at least 15 mm less than the first Z-direction caliper Z1, under a compressive load of less than 100 grams, and preferably less than 50 grams. A sanitary napkin 20 having a lifting member 100 can also have a Z-direction caliper of less than 10 mm under a compressive load of 90 grams, so that the sanitary napkin 20 can be compressed to take on a relatively thin, flat shape (such as when the wearer sits down) without causing the wearer discomfort.

The Z-direction calipers Z1 and Z2, and the corresponding Z-direction compressive loading are measured using the following procedure with an INSTRON Model 4502 tensile test machine manufactured by the Instron Engineering Corp. of Canton, Mass. The sanitary napkins 20 to be tested should be conditioned for about 2 hours in a room at between 71 and 75 degree Fahrenheit and 48 to 52 percent relative humidity prior to testing.

The tensile test machine is equipped with a 100 gram load cell. The sanitary napkin 20 is supported, topsheet 22 facing upward, with the garment facing surface 25 of the backsheet 24 facing downward and resting on a horizontal surface of a 6 inch diameter plate attached to the stationary jaw of the tensile test machine. A 1.0 inch diameter horizontal compression foot is attached to the moving crosshead of the tensile test machine to face the topsheet 22 of the sanitary napkin 20. The compression foot is positioned over the longitudinal centerline 29 of the sanitary napkin about midway along the length of the lifting member 100. The initial Z-direction spacing between the stationary plate surface and the compression foot is greater than the unloaded Z-direction caliper of the of the sanitary napkin 20, and is at least 30 mm or greater. The compression foot is then advanced toward the stationary plate surface at a constant rate (crosshead speed) of 25 inches per minute. The force measured by the load cell for a given spacing between the compression foot and the stationary plate surface is recorded on a strip chart recorder at a chart speed of 250 inches per minute. The spacing between the compression foot and the stationary plate surface at a given load corresponds to the Z-direction caliper of the sanitary napkin 20 at that load. When the spacing between the compression foot and the stationary plate surface is reduced to 3 mm, the direction of travel of the compression foot is reversed to retract from the stationary plate surface at a speed of 25 inches per minute.

The data in Table 1 were obtained using the above procedure to measure the Z-direction caliper of three sanitary napkins 20, each having a lifting member 100 according to the present invention.

**TABLE 1**

| Z-DIRECTION CALIPER AND LOADING | | | | | |
|---|---|---|---|---|---|
| Measurement | | Average | S.D. | Min. | Max. |
| A. | Caliper at 2 gm load: | 27.3mm | 1.24 | 25.5mm | 28.9mm |
| B. | Caliper at 2 gm unload: | 23.9mm | 1.03 | 23.4mm | 25.7mm |
| C. | Caliper reduced 10mm from A | 17.3mm | 1.24 | 15.5mm | 18.9mm |
| D. | Force at Caliper C | 27.7 gm | 3.9 | 25.5gm | 34.5gm |
| E. | Caliper reduced 15mm from A | 12.3mm | 1.24 | 10.5mm | 13.9mm |
| F. | Force at Caliper E | 44.1gm | 14.21 | 34.0gm | 67.0gm |
| G. | Caliper at 90 gm load | 9.5mm | 0.31 | 9.1mm | 9.8mm |

Table 1 lists the average, standard deviation, minimum, and maximum of measurements A-G for three sanitary napkins 20 having a lifting member 100. Measurement A is the Z-direction caliper of the sanitary napkin 20 at a load cell reading of 2 grams, as the compression foot is advancing toward the stationary plate surface. The caliper at a load of 2 grams is essentially the caliper Z1 of an unloaded sanitary napkin. Measurement B is the Z-direction caliper of the sanitary napkins 20 at a load cell reading of 2 grams, as the compression foot is retracting from the stationary plate surface, and shows that lifting member 100 substantially restores the original, unloaded caliper of the sanitary napkin 20 upon removal of the Z-direction loading.

Measurement C corresponds to a second caliper Z2 which is 10 mm less than the caliper Z1 at 2 grams load, and measurement D is the Z-direction compressive force at the caliper C. Measurement E corresponds to a second caliper Z2 which is 15 mm less than the caliper Z 1 at 2 grams load, and measurement F is the Z-direction compressive force measured at the caliper E. Measurement G is the Z-direction caliper of the sanitary napkin 20 when the Z-direction compressive force is equal to 90 grams.

As shown in Figure 5, the core 26 can be segmented along the longitudinal axis 29 to comprise a plurality of core segments 26A, 26B, and 26C which are independently displaceable in the Z-direction. The adjacent core segments, such as core segments 26A,B and 26B,C can be joined by laterally extending hinge lines 27. The hinge lines 27 can include, but are not limited to lines of embossment or compaction, creases, score lines or pre-fold lines. Alternatively, adjacent core segments can be unattached, and indirectly joined to each other by the topsheet 22.

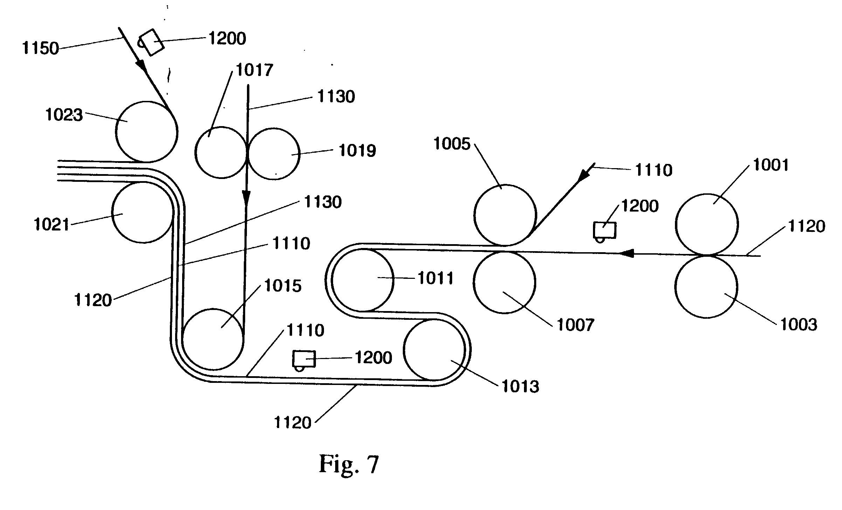

Figure 7 illustrates an apparatus for forming a pleated lifting member 100 and joining the lifting member 100 to another component of the sanitary napkin 20. In Figure 7, three continuously fed pieces of material are designated as components 1100, 1120, and 1130. By way of example component 1100 can correspond to the first element 110, component 1120 can correspond to the elastic second element 120, and component 1130 can correspond to the elastic third element 130. The components 1120 and 1130 can be bonded to the component 1100 at spaced apart locations along the length of the component 1100, such as with an adhesive. The discussion below refers to adhesive bonding of the components 1100, 1200 and 1300, but it will be understood that other bonding methods can be used. For instance, the components can be thermally or ultrasonically bonded together. Alternatively, the components can be mechanically bonded together in a pressure biased nip between a relief patterned cylinder and an anvil cylinder according to the teachings of U.S. Patent 4,919,738 issued April 24, 1990 to Ball et al.

The component 1120 is carried at a first web speed through a first nip formed between a first pair of compression rolls 1001,1003 rotating at a first rotational speed. An adhesive applicator 1200 applies adhesive to a side of the component 1120 at spaced apart locations after the component 1120 exits the nip formed by rolls 1001,1003. A suitable adhesive is Findley Adhesive H2031 available from the Findley Adhesive Company of Elmgrove, Wisconsin.

The component 1120, along with component 1110, is then directed into a second nip formed by a second pair of compression rolls 1005,1007. The component 1120 is pressed into engagement with the component 1110 by the second pair of rolls 1005,1007 to adhesively bond the components 1120 and 1110 together at spaced apart locations. The nip rolls 1005,1007 can be chilled to prevent buildup of the adhesive.

The surface speed of the rolls 1005,1007 carries the components 1120 and 1110 through the second nip at a second web speed greater than the first web speed at which the component 1120 is carried through the first nip. This web speed differential stretches the component 1120, and thereby differentially elongates the component 1120 relative to the component 1110 prior to intermittently joining the component 1120 to the component 1110. For example, if the compression rolls have the same diameter, the second pair of compression rolls 1005,1007 can be rotated at a second rotational speed which is greater than the first rotational speed of the rolls 1001,1003.

The intermittently joined components 1110 and 1120 exit the second nip and are carried around first and second reversing rolls 1011 and 1013 in a generally serpentine path. An adhesive applicator 1200 then applies adhesive to spaced apart locations on a side of the component 1110 not joined to the component 1120. The web speed around the rolls 1011, and 1013 can be maintained equal to the second web speed to maintain the tensile elongation in the component 1120.

The intermittently joined components 1110 and 1120, along with the component 1130, are then directed around a roll 1015 to press the component 1130 into engagement with side of the component 1110 to which adhesive is intermittently applied. The component 1130 is thereby joined to the component 1110 at spaced apart locations. The web speed around the roll 1015 can be maintained equal to the second web speed to maintain the tensile elongations in the component 1120.

Prior to being joined to the component 1110, the component 1130 can be directed at a third web speed through a third nip formed by a third pair of compression rolls 1017, 1019 rotating at a third rotational speed. The third web speed can be less than the second web speed at which the components 1110,1120, and 1130 are carried around the roll 1015. This web speed differential stretches the component 1130, so that the component 1130 is differentially elongated relative to the component 1110. If the third web speed is equal to the first web speed, the percent elongation of the component 1130 will be approximately equal to the percent elongation of the component 1120. Alternatively, if the third web speed is less than (or greater than) the first web speed, the percent elongation of the component 1130 will be greater than (or less than) the percent elongation of the component 1120. Such a difference in percentage elongation between the components 1120 and 1130 can promote curvature along the length of the sanitary napkin 20. For example, if component 1130 corresponds to the elastic element 120, and is to be joined to the core 26, the component 1130 can be elongated about 1 to 5 percent more than the component 1120 to bow the sanitary napkin 20 to have upraised forward and rear ends 30A and 30B.

The combined components 1110, 1120, and 1130, along with a component 1150, can be directed at a fourth web speed through a fourth nip formed by a fourth pair of compression rolls 1021,1023. The component 1150 can comprise a web of material for forming the backsheet 24, or alternatively, a web of material forming the laminate of the topsheet 22 and the core 26. An adhesive applicator 1200 can apply adhesive to a side of the component 1150 prior to engagement of the component 1150 with the component 1130 in the fourth nip. The assembly of components 1110, 1120, 1130, and 1150 can be combined in later operations with other sanitary napkin components to form a completed sanitary napkin 20.

The tension in the stretched components 1120 and/or 1130 can be relaxed, such as by reducing the web speed before or after the components 1110,1120 and 1130 are combined with the component 1150 in the fourth nip. Once the tension in the stretched components is relaxed, the stretched components will elastically contract relative to the component 1110, and thereby gather the component 1110 to form pleats 115.

A lifting member 100 having pleats 115 with a Z-direction height that varies along the length of the lifting member can be formed by varying one or more of the web speeds with time. The elongation of the component 1120 relative to the component 1110 will then vary along the length of the intermittently joined components. Once the tension in the component 1120 is relaxed, the elastic contraction of the component 1120 relative to the component 1110 will vary along the length of the intermittently joined components, thereby forming pleats 115 having different Z-direction heights.

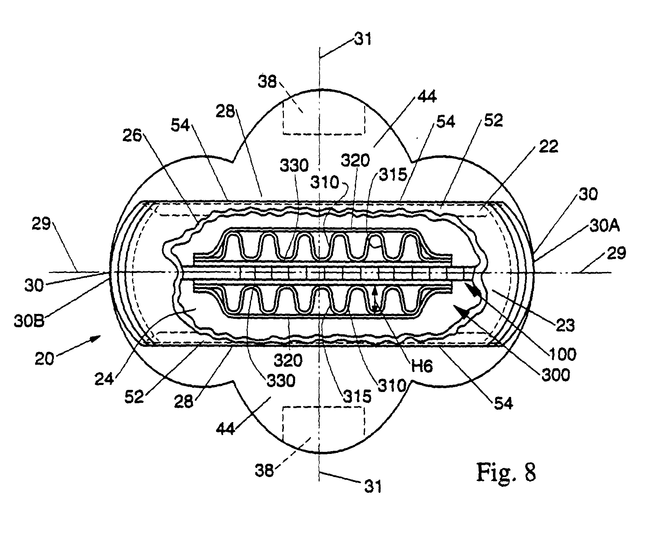

Figure 8 shows a sanitary napkin 20 having a pleated lifting member 100 having Z-direction pleats, and two lateral displacement members 300 for providing elastic displacement of the longitudinal ends 28 of the sanitary napkin 20 in a direction generally parallel to the lateral centerline 31. The lateral displacement members 300 have lateral direction pleats 315 having a lateral pleat height H6, measured generally parallel to the lateral centerline 31. The lateral displacement members 300 accommodate lateral compression of the sanitary napkin 20 between the wearer's thighs. When the lateral compressive loads exerted by the wearer's thighs are relaxed, the lateral spacing member helps to restore the lateral spacing between the longitudinal ends 28, and thereby maintains an effective lateral width of the sanitary napkin 20 for receiving body exudates.

The lateral displacement members 300 can be formed in a manner similar to which the lifting member 100 is formed. Each lateral displacement member 300 can have a first pleated element 310 and elastic second and third elements 320 and 330. One or both of the second and third elements 320 and 330 can gather the first element 310 along fold lines generally perpendicular to the longitudinal and lateral axes 29 and 31 to form the pleats 315 extending laterally outward from the longitudinal centerline 29. The lateral displacement members 300 are preferably disposed intermediate the topsheet 22 and the backsheet 24, and more preferably are disposed intermediate the core 26 and the backsheet 24.

## Claims

1. A disposable absorbent article (20) having a longitudinal centerline (29) and longitudinal ends (28) joining first and second lateral ends (30), the absorbent article (20) comprising:
a liquid pervious topsheet (22);
a liquid impervious backsheet (24),
an absorbent core (26) disposed intermediate the topsheet (22) and the backsheet (24); and
a longitudinally extending lifting member (100) disposed intermediate the topsheet (22) and the backsheet (24), characterized in that said lifting member (100) comprising a plurality of pleats (115) along the length of the lifting member (100), the pleats (115) having a Z-direction height for providing Z-direction displacement of a portion of the topsheet (22) relative to the backsheet (24).

2. The disposable absorbent (20) article of Claim 1. characterized in that the lifting member (100) extends along the longitudinal centerline (29) of the absorbent article (20).

3. The disposable absorbent article (20) of Claim 2 characterized in that the Z-direction height of the pleats (115) varies along the longitudinal axis (29) of the absorbent article (20).

4. The disposable absorbent article (20) of Claim 1 characterized in that the lifting member (100) convexly shapes a portion of the body facing surface of the topsheet (22) along the longitudinal centerline (29) of the disposable absorbent article (20).

5. The disposable absorbent article (20) of Claim 1 characterized in that the lifting member (100) is disposed intermediate the absorbent core (26) and the backsheet (24).

6. The disposable absorbent article (20) of Claim 1 characterized in that the lifting member (100) comprises a pleated first element (110) and a second element (120) joined to the first element (110) at spaced apart locations along the length of the first element (110), and characterized in that elastic contraction of the second element (120) relative to the first element (110) gathers the first element (110) to form pleats (115) in the first element (110).

7. The disposable absorbent article (20) of Claim 6 characterized in that the second element (120) comprises an elastic element.

8. The disposable absorbent article (20) of Claim 7 characterized in that differential elastic contraction of the second element (120) along the length of the second element (120) gathers the first element (110) to form pleats (115) having varying Z-direction heights.

9. The disposable absorbent article (20) of Claim 7 characterized in that the second element (120) is disposed intermediate the first element (110) and the absorbent core (26).

10. The disposable absorbent article (20) of Claim 9 characterized in that the second element (120) is joined to the absorbent core (24).

11. The disposable absorbent article (20) of Claim 7 further comprising an elastic third element (130), and characterized in that the first element (110) is joined to the third element (130) at spaced apart locations along the length of the first element (110), and characterized in that the pleats (115) of the first element (110) extend between the second and third elements (120, 130).

12. The disposable absorbent article (20) of Claim 11 characterized in that one of the second and third members (120, 130) is joined to the absorbent core (26) and the other of the second and third members (120, 130) is joined to the backsheet (24).

13. The disposable absorbent article (20) of Claim 1 characterized in that the lifting member (110) is nonabsorbent.

14. The disposable absorbent article (20) of Claim 1 characterized in that the Z-direction height of the pleats (115) varies along the length of the lifting member (100).

15. The disposable absorbent article (20) of Claim 14 characterized in that the Z-direction height of the pleats (115) has a first value along a center portion of the lifting member (100) and a second lower value in forward and rearward end portions of the lifting member (100).

16. The disposable absorbent article (20) of Claim 14 characterized in that the Z-direction height of the pleats (115) is tapered to have a maximum value rearward of a lateral centerline (28) of the disposable absorbent article (20).

17. A disposable absorbent article (20) according to any of the preceding claims wherein said lifting member (100) comprises:
a longitudinally extending first element (110) disposed intermediate the topsheet (22) and the backsheet (24); and
a second elastic element (120) joined to the first element (110) at spaced apart locations along the length of the first element (110);
characterized in that the second elastic element (120) elstically gathers the first element (110) to form a plurality of pleats (115) along the length of the first element (110).

18. A disposable absorbent article (20) according to claim 17 wherein said lifting member (100) is disposed intermediate the absorbent core (20) and the backsheet (24).

19. The absorbent article (20) of Claim 18 characterized in that the second elastic element (120) is disposed intermediate the first element (110) and the absorbent core (26).

20. The absorbent article (20) of Claim 18 further comprising a third elastic element (130) joined to the first element (110) at spaced apart locations along the length of the first elastic element (110), and characterized in that the pleats (115) of the first element (110) extend between the second and third elastic elements (120, 130).

21. A disposable absorbent article (20) according to any of the preceding claims wherein said pleats have a Z-direction height of at least 10 millimeters.

22. The absorbent article (20) of Claim 21 having a first Z-direction caliper under a Z-direction compressive load of 2 grams, and a second Z-direction caliper under a compressive load less than 50 grams, characterized in that the second Z-direction caliper is at least 10 millimeters less than the first Z-direction caliper.

23. The absorbent article (20) of Claim 22 having a second Z-direction caliper under a compressive load less than 30 grams, characterized in that the second Z-direction caliper is at least 10 millimeters less than the first Z-direction caliper.

24. The absorbent article (20) of Claim 21 having a first Z-direction caliper under a Z-direction compressive load of 2 grams, and a second Z-direction caliper under a compressive load less than 100 grams, characterized in that the second Z-direction caliper is at least 15 millimeters less than the first Z-direction caliper.

25. The absorbent article (20) of Claim 24 having a second Z-direction caliper under a compressive load less than 50 grams, characterized in that the second Z-direction caliper is at least 15 millimeters less than the first Z-direction caliper.

26. The absorbent article (20) of Claim 1 comprising at least one pleated element (110) having laterally extending pleats (115).

27. The absorbent article (20) of Claim 1 wherein the lifting member (100) has a wet caliper reduction of no more than about 8 percent.

28. The absorbent article (20) of Claim 1 wherein the lifting member (100) has a wet caliper reduction which is no more than about 20 percent greater than the dry caliper reduction of the lifting member (100).

## Patentansprüche

1. Wegwerfbarer absorbierender Artikel (20) mit einer longitudinalen Zentrallinie (29) und longitudinalen Enden (28), die erste und zweite seitliche Enden (30) verbinden, wobei der absorbierende Artikel (20) umfaßt:
eine flüssigkeitsdurchlässige Decklage (22);
eine flüssigkeitsundurchlässige Außenlage (24),
einen absorbierenden Kern (26), der zwischen der Decklage (22) und der Außenlage (24) angeordnet ist; und
ein sich in Längsrichtung erstreckendes Anhebeteil (100), das zwischen der Decklage (22) und der Außenlage (24) angeordnet ist, wobei das Anhebeteil (100) eine Vielzahl von Falten (115) entlang der Länge des Anhebeteils (100) aufweist, wobei die Falten (115) eine Höhe in Z-Richtung aufweisen, um eine Verschiebung in Z-Richtung eines Abschnittes der Decklage (22) relativ zur Außenlage (24) zu schaffen.

2. Wegwerfbarer absorbierender Artikel (20) nach Anspruch 1, **dadurch gekennzeichnet, daß** das Anhebeteil (100) sich entlang der longitudinalen Zentrallinie (29) des absorbierenden Artikels (20) erstreckt.

3. Wegwerfbarer absorbierender Artikel (20) nach Anspruch 2, **dadurch gekennzeichnet, daß** die Höhe in Z-Richtung der Falten (115) entlang der Longitudinalachse (29) des absorbierenden Artikels (20) variiert.

4. Wegwerfbarer absorbierender Artikel (20) nach Anspruch 1, **dadurch gekennzeichnet, daß** das Anhebeteil (100) auf konvexe Weise einen Abschnitt der dem Körper zugewandten Oberfläche der Decklage (22) entlang der longitudinalen Zentrallinie (29) des wegwerfbaren absorbierenden Artikels (20) ausbildet.

5. Wegwerfbarer absorbierender Artikel (20) nach Anspruch 1, **dadurch gekennzeichnet, daß** das Anhebeteil (100) zwischen dem absorbierenden Kern (26) und der Außenlage (24) angeordnet ist.

6. Wegwerfbarer absorbierender Artikel (20) nach Anspruch 1, **dadurch gekennzeichnet, daß** das Anhebeteil (100) ein gefaltetes erstes Element (110) und ein zweites Element (120) aufweist, das mit dem ersten Element (110) an voneinander beabstandeten Stellen entlang der Länge des ersten Elementes (110) verbunden ist, und **dadurch gekennzeichnet, daß** eine elastische Kontraktion des zweiten Elementes (120) relativ zum ersten Element (110) das erste Element (110) rafft, um Falten (115) in dem ersten Element (110) zu bilden.

7. Wegwerfbarer absorbierender Artikel (20) nach Anspruch 6, **dadurch gekennzeichnet, daß** das zweite Element (120) ein elastisches Element umfaßt.

8. Wegwerfbarer absorbierender Artikel (20) nach Anspruch 7, **dadurch gekennzeichnet, daß** eine unterschiedliche elastische Kontraktion des zweiten Elementes (120) entlang der Länge des zweiten Elementes (120) das erste Element (110) so rafft, daß Falten (115) mit variierender Höhe in Z-Richtung gebildet werden.

9. Wegwerfbarer absorbierender Artikel (20) nach Anspruch 7, **dadurch gekennzeichnet, daß** das zweite Element (120) zwischen dem ersten Element (110) und dem absorbierenden Kern (26) angeordnet ist.

10. Wegwerfbarer absorbierender Artikel (20) nach Anspruch 9, **dadurch gekennzeichnet, daß** das zweite Element (120) mit dem absorbierenden Kern (26) verbunden ist.

11. Wegwerfbarer absorbierender Artikel (20) nach Anspruch 7, weiterhin umfassend ein elastisches drittes Element (130) und **dadurch gekennzeichnet, daß** das erste Element (110) mit dem dritten Element (130) an voneinander entlang der Länge des ersten Elementes (110) beabstandeten Stellen verbunden ist, und **dadurch gekennzeichnet**, daß die Falten (115) des ersten Elementes (110) sich zwischen dem zweiten und dritten Element (120, 130) erstrecken.

12. Wegwerfbarer absorbierender Artikel (20) nach Anspruch 11, **dadurch gekennzeichnet, daß** eines der zweiten und dritten Elemente (120, 130) mit dem absorbierenden Kern (26) und das andere der zweiten und dritten Elemente (120, 130) mit der Außenlage (24) verbunden sind.

13. Wegwerfbarer absorbierender Artikel (20) nach Anspruch 1, **dadurch gekennzeichnet, daß** das Anhebeteil (110) nicht-absorbierend ist.

14. Wegwerfbarer absorbierender Artikel (20) nach Anspruch 1, **dadurch gekennzeichnet, daß** die Höhe in Z-Richtung der Falten (115) entlang der Länge des Anhebeteils (100) variiert.

15. Wegwerfbarer absorbierender Artikel (20) nach Anspruch 14, **dadurch gekennzeichnet, daß** die Höhe in Z-Richtung der Falten (115) einen ersten Wert entlang eines Zentralabschnittes des Anhebeteils (100) und einen zweiten, geringeren Wert in vorderen und rückwärtigen Endabschnitten des Anhebeteils (100) aufweist.

16. Wegwerfbarer absorbierender Artikel (20) nach Anspruch 14, **dadurch gekennzeichnet, daß** die Höhe in Z-Richtung der Falten (115) abgeschrägt ist, um einen maximalen Wert hinter einer lateralen Zentrallinie (28) des wegwerfbaren absorbierenden Artikels (20) aufzuweisen.

17. Wegwerfbarer absorbierender Artikel (20) nach einem der vorgenannten Ansprüche, wobei das Anhebeteil (100) umfaßt
ein sich in Längsrichtung erstreckendes erstes Element (110), das zwischen der Decklage (22) und der Außenlage (24) angeordnet ist; und ein zweites elastisches Element (120), das mit dem ersten Element (110) an voneinander beabstandeten Stellen entlang der Länge des ersten Elementes (110) verbunden ist;
**dadurch gekennzeichnet**, daß das zweite elastische Element (120) das erste Element (110) elastisch rafft, um eine Vielzahl von Falten (115) entlang der Länge des ersten Elementes (100) zu bilden.

18. Wegwerfbarer absorbierender Artikel (20) nach Anspruch 17, wobei das Anhebeteil (100) zwischen dem absorbierenden Kern (26) und der Außenlage (24) angeordnet ist.

19. Wegwerfbarer absorbierender Artikel (20) nach Anspruch 18, **dadurch gekennzeichnet, daß** das zweite elastische Element (120) zwischen dem ersten Element (110) und dem absorbierenden Kern (26) angeordnet ist.

20. Wegwerfbarer absorbierender Artikel (20) nach Anspruch 18, ferner umfassend ein drittes elastisches Element (130), das mit dem ersten Element (110) an voneinander beabstandeten Stellen entlang der Länge des ersten elastischen Elementes (110) verbunden ist und **dadurch gekennzeichnet, daß** die Falten (115) des ersten Elementes (110) sich zwischen dem zweiten und dritten elastischen Element (120, 130) erstrecken.

21. Wegwerfbarer absorbierender Artikel (20) nach einem der vorgenannten Ansprüche, wobei die Falten eine Höhe in Z-Richtung von zumindest 10 mm aufweisen.

22. Absorbierender Artikel (20) nach Anspruch 21 mit einer ersten Stärke in Z-Richtung unter einer Kompressionsbelastung in Z-Richtung von 2 Gramm und einer zweiten Stärke in Z-Richtung unter einer Kompressionsbelastung von weniger als 50 Gramm, **dadurch gekennzeichnet, daß** die zweite Stärke in Z-Richtung zumindest 10 mm geringer ist als die erste Stärke in Z-Richtung.

23. Absorbierender Artikel (20) nach Anspruch 22 mit einer zweiten Stärke in Z-Richtung unter einer Kompressionsbelastung von weniger als 30 Gramm, **dadurch gekennzeichnet, daß** die zweite Stärke in Z-Richtung zumindest 10 mm geringer ist als die erste Stärke in Z-Richtung.

24. Absorbierender Artikel (20) nach Anspruch 21 mit einer ersten Stärke in Z-Richtung unter einer Kompressionsbelastung in Z-Richtung von 2 Gramm und einer zweiten Stärke in Z-Richtung unter einer Kompressionsbelastung von weniger als 100 Gramm, **dadurch gekennzeichnet, daß** die zweite Stärke in Z-Richtung zumindest 15 mm geringer ist als die erste Stärke in Z-Richtung.

25. Absorbierender Artikel (20) nach Anspruch 24 mit einer zweiten Stärke in Z-Richtung unter einer Kompressionsbelastung von weniger als 50 Gramm, **dadurch gekennzeichnet, daß** die zweite Stärke in Z-Richtung zumindest 15 mm geringer als die erste Stärke in Z-Richtung ist.

26. Absorbierender Artikel (20) nach Anspruch 1, umfassend zumindest ein plissiertes Element (110) mit sich seitlich erstreckenden Falten (115).

27. Absorbierender Artikel (20) nach Anspruch 1, wobei das Anhebeteil (100) eine Naß-Stärke-Reduktion von nicht mehr als etwa 8 % aufweist.

28. Absorbierender Artikel (20) nach Anspruch 1, wobei das Anhebeteil (100) eine Naß-Stärke-Reduktion aufweist, die nicht mehr als etwa 20 % größer als die Trocken-Stärke-Reduktion des Anhebeteils (100) ist.

## Revendications

1. Article absorbant jetable (20) comportant une ligne médiane longitudinale (29) et des extrémités longitudinales (28) réunissant des première et deuxième extrémités latérales (30), l'article absorbant (20) comprenant:
une feuille de dessus (22) perméable aux liquides ;
une feuille de fond (24) imperméable aux liquides ;
une âme absorbante (26) placée entre la feuille de dessus (22) et la feuille de fond (24) ; et un élément d'élévation (100) s'étendant longitudinalement placé entre la feuille de dessus (22) et la feuille de fond (24), caractérisé en ce que ledit élément d'élévation (100) comprend une pluralité de plis (115) suivant la longueur de l'élément d'élévation (100), les plis (115) ayant une hauteur de direction Z afin d'assurer un déplacement de direction Z d'une partie de la feuille de dessus (22) par rapport à la feuille de fond (24).

2. Article absorbant jetable (20) selon la revendication 1, caractérisé en ce que l'élément d'élévation (100) s'étend le long de la ligne médiane longitudinale (29) de l'article absorbant (20).

3. Article absorbant jetable (20) selon la revendication 2, caractérisé en ce que la hauteur de direction Z des plis (115) varie le long de l'axe longitudinal (29) de l'article absorbant (20).

4. Article absorbant jetable (20) selon la revendication 1, caractérisé en ce que l'élément d'élévation (100) conforme de manière convexe une partie de la surface faisant face au corps de la feuille de dessus (22) le long de la ligne médiane longitudinale (29) de l'article absorbant jetable (20).

5. Article absorbant jetable (20) selon la revendication 1, caractérisé en ce que l'élément d'élévation (100) est placé entre l'âme absorbante (26) et la feuille de fond (24).

6. Article absorbant jetable (20) selon la revendication 1, caractérisé en ce que l'élément d'élévation (100) comprend un premier élément plissé (110) et un deuxième élément (120) réuni au premier élément (110) en des emplacements espacés situés suivant la longueur du premier élément (110), et caractérisé en ce que la contraction élastique du deuxième élément (120) par rapport au premier élément (110) fronce le premier élément (110) afin de former des plis (115) dans le premier élément (110).

7. Article absorbant jetable (20) selon la revendication 6, caractérisé en ce que le deuxième élément (120) comprend un élément élastique.

8. Article absorbant jetable (20) selon la revendication 7, caractérisé en ce qu'une contraction élastique différentielle du deuxième élément (120) suivant la longueur du deuxième élément (120) fronce le premier élément (110) afin de former des plis (115) ayant des hauteurs variables de direction Z.

9. Article absorbant jetable (20) selon la revendication 7, caractérisé en ce que le deuxième élément (120) est placé entre le premier élément (110) et l'âme absorbante (26).

10. Article absorbant jetable (20) selon la revendication 9, caractérisé en ce que le deuxième élément (120) est réuni à l'âme absorbante (24).

11. Article absorbant jetable (20) selon la revendication 7, comprenant, en outre, un troisième élément élastique (130), et caractérisé en ce que le premier élément (110) est réuni au troisième élément (130) en des emplacements eapacés situés suivant la longueur du premier élément (110), et caractérisé en ce que les plis (115) du premier élément (110) s'étendent entre les deuxième et troisième éléments (120, 130).

12. Article absorbant jetable (20) selon la revendication 11, caractérisé en ce que l'un des deuxième et troisième éléments (120, 130) est réuni à l'âme absorbante (26), et l'autre des deuxième et troisième éléments (120, 130) est réuni à la feuille de fond (24).

13. Article absorbant jetable (20) selon la revendication 1, caractérisé en ce que l'élément d'élévation (100) n'est pas absorbant.

14. Article absorbant jetable (20) selon la revendication 1, caractérisé en ce que la hauteur de direction Z des plis (115) varie suivant la longueur de l'élément d'élévation (100).

15. Article absorbant jetable (20) selon la revendication 14, caractérisé en ce que la hauteur de direction Z des plis (115) a une première valeur le long d'une partie centrale de l'élément d'élévation (100) et une deuxième valeur inférieure dans les parties d'extrémité avant et arrière de l'élément d'élévation (100).

16. Article absorbant jetable (20) selon la revendication 14, caractérisé en ce que la hauteur de direction Z des plis (115) est effilée afin d'avoir une valeur maximale en arrière d'une ligne médiane latérale (25) de l'article absorbant (20).

17. Article absorbant jetable (20) selon l'une quelconque des revendications précédentes, dans lequel ledit élément d'élévation (100) comprend un premier élément (110) s'étendant longitudinalement disposé entre la feuille de dessus (22) et la feuille de fond (24), et un deuxième élément élastique (120) réuni au premier élément élastique (110) en des emplacements espacés situés suivant la longueur du premier élément (110). caractérisé en ce que le deuxième élément élastique (120) fronce élastiquement le premier élément (110) afin de former une pluralité de plis (115) suivant la longueur du premier élément (110).

18. Article absorbant jetable (20) selon la revendication 17, dans lequel ledit élément d'élévation (100) est placé entre l'âme absorbante (26) et la feuille de fond (24).

19. Article absorbant (20) selon la revendication 18, caractérisé en ce que le deuxième élément élastique (120) est placé entre le premier élément (110) et l'âme absorbante (26).

20. Article absorbant (20) selon la revendication 18, comprenant, en outre, un troisième élément élastique (130) réuni au premier élément (110) en des emplacements espacés situés suivant la longueur du premier élément élastique (110), et caractérisé en ce que les plis (115) du premier élément (110) s'étendent entre les deuxième et troisième éléments élastiques (120, 130).

21. Article absorbant jetable (20) selon l'une quelconque des revendications précédentes, dans lequel lesdits plis ont une hauteur de direction Z d'au moins 10 millimètres.

22. Article absorbant (20) selon la revendication 21, ayant un premier calibre de direction Z sous une charge de compression de direction Z de 2 grammes, et un deuxième calibre de direction Z sous une charge de compression inférieure à 50 grammes, caractérisé en ce que le deuxième calibre de direction Z est inférieur au moins de 10 millimètres au premier calibre de direction Z.

23. Article absorbant (20) selon la revendication 22, ayant un deuxième calibré de direction Z sous une charge de compression inférieure à 30 grammes, caractérisé en ce que le deuxième calibre de direction Z est inférieur au moins de 10 millimètres au premier calibre de direction Z.

24. Article absorbant (20) selon la revendication 21, ayant un premier calibre de direction Z sous une charge de compression de direction Z de 2 grammes, et un deuxième calibre de direction Z sous une charge de compression inférieure à 100 grammes, caractérisé en ce que le deuxième calibre de direction Z est inférieur au moins de 15 millimètres au premier calibre de direction Z.

25. Article absorbant (20) selon la revendication 24, ayant un deuxième calibre de direction Z sous une charge de compression inférieure à 50 grammes, caractérisé en ce que le deuxième calibre de direction Z est inférieur au moins de 15 millimètres au premier calibre de direction Z.

26. Article absorbant (20) selon la revendication 1, comprenant au moins un élément plissé (110) comportant des plis (115) s'étendant latéralement.

27. Article absorbant (20) selon la revendication 1, dans lequel l'élément d'élévation (100) a une réduction de calibre à l'état humide ne dépassant pas environ 8 pour-cent.

28. Article absorbant (20) selon la revendication 1, dans lequel l'élément d'élévation (100) a une réduction de calibre à l'état humide qui n'est pas supérieur de plus de 20 pour-cent environ à la réduction de calibre à l'état sec de l'élément d'élévation (100).
